# EUROPÄISCHE PATENTANMELDUNG

(11) **EP 0 943 353 A2**
(43) Veröffentlichungstag der Anmeldung: **22.09.1999**
(21) Anmeldenummer: 99105202.8
(22) Anmeldetag: 12.03.1999
(51) Int. Cl.: A61M 16/00, A61M 16/20

(54) **Verfahren zum Betrieb eines Beatmungsgerätes und Vorrichtung zur Beatmung**

(30) Priorität: 20.03.1998 DE 19812392
(71) Anmelder: GOTTLIEB WEINMANN GERÄTE FÜR MEDIZIN UND ARBEITSSCHUTZ GMBH & CO., D-22525 Hamburg (DE)
(72) Erfinder: Newzella, Martin, 23843 Bad Oldesloe (DE); Schulte, Andrè, 22756 Hamburg (DE); Delin, Heinz, 25451 Quickborn (DE)
(74) Vertreter: Klickow, Hans-Henning

(57) **Zusammenfassung**

Das Verfahren und die Vorrichtung dienen zur Durchführung einer Patientenbeatmung bei mobilen Versorgungen. Es wird sensorisch mindestens ein Atmungsparameter des Patienten erfaßt und zur Gerätesteuerung derart ausgewertet, daß im wesentlichen eine detektierte Atmungsfehlfunktion substituiert wird. Darüber hinaus wird eine eigene Atmungsaktivität des Patienten berücksichtigt. Zur Unterstützung eines mobilen Einsatzes wird eine Betriebsenergie teilweise von einer Druckgasspeisung bereitgestellt. Zur Erreichung einer schnellen Betriebsfähigkeit sind unterschiedliche Parametersätze zur Definition unterschiedlicher Betriebsmodi voreinstellbar. Eine Parametereinstellung kann über ein Proportionalventil in Piezo-Technik durchgeführt werden, das sowohl bei mobilen als auch bei stationären Beatmungsgeräten verwendbar ist.

## Beschreibung

Die Erfindung betrifft ein Verfahren zum Betrieb eines Beatmungsgerätes, das bei einer mobilen Versorgung eines Patienten angewendet wird.

Die Erfindung betrifft darüber hinaus eine Vorrichtung zur Durchführung einer Beatmung eines Patienten, die als eine mobile Einrichtung ausgebildet ist und die mindestens ein Einstellelement zur Vorgabe mindestens eines Bedienparameters aufweist.

Desweiteren betrifft die Erfindung eine Vorrichtung zur Durchführung einer Beatmung eines Patienten, die eine Atemgasversorgung, ein Kopplungselement zum Anschluß an den Patienten sowie mindestens ein Einstellelement zur Beeinflussung mindestens eines Beatmungsparameters aufweist.

Derartige Verfahren und Vorrichtungen werden in unterschiedlichen Gestaltungen im medizinischen Bereich verwendet. Grundsätzlich wird zwischen Anwendungen im mobilen Bereich sowie im stationären Bereich unterschieden. Mobile Anwendungen finden sich insbesondere im prähospitalen Einsatz bei Primärtransporten, Sekundärtransporten sowie bei innerklinischen Transporten. Weitere Anwendungen können im quasistationären Bereich gegeben sein, bei dem bekannte stationäre klinische Vorrichtungen aus wirtschaftlichen oder logistischen Überlegungen nicht zum Einsatz kommen können.

Stationäre Anwendungen sind hauptsächlich im klinischen Bereich gegeben, um eine qualitativ hochwertige Patientenversorgung zu gewährleisten.

Die bislang bekannten mobilen Beatmungsvorrichtungen, die überwiegend bei der Durchführung von Tranportbeatmungen verwendet werden, funktionieren im wesentlichen entsprechend einer getakteten Luftpumpe, die in vorgegeben zeitlichen Intervallen ein vorgegebenes Atmungsvolumen in die Lungen des Patienten einleitet und in zwischenliegenden Entspannungsphasen ein Entweichen der Atmungsluft zuläßt bzw. dies aktiv unterstützt. Ggf. vorhandene Restfähigkeiten des Patienten zur eigenständigen Durchführung der Atmungsvorgänge bleiben hierbei in der Regel unberücksichtigt. Dies hat zur Folge, daß die Restatmungsfunktion des Patienten bei der Durchführung der Transportbeatmung weiter abnehmen kann.

Aufgabe der vorliegenden Erfindung ist es, ein Verfahren der einleitend genannten Art derart zu verbessern, daß eine Beeinträchtigung von Atmungsfunktionen des Patienten vermieden wird.

Diese Aufgabe wird erfindungsgemäß dadurch gelöst, daß sensorisch mindestens ein Atmungsparameter des Patienten erfaßt und zur Gerätesteuerung derart ausgewertet wird, daß im wesentlichen eine detektierte Atmungsfehlfunktion substituiert und eine eigene Atmungsaktivität des Patienten berücksichtigt wird und daß eine Betriebsenergie mindestens teilweise von einer Druckgasspeisung bereitgestellt wird.

Eine weitere Variante zur Lösung der vorliegenden Aufgabe besteht darin, daß ein Volumenstrom des Atemgases und ein Beatmungsdruck derart aufeinander abgestimmt werden, daß bei zunehmender Lungenfüllung des Patienten eine Reduzierung des Volumenstromes zur Druckbegrenzung durchgeführt wird.

Weitere Aufgabe der vorliegenden Erfindung ist es, eine Vorrichtung der einleitend genannten Art derart zu konstruieren, daß eine einfache Bedienbarkeit unterstützt wird.

Diese Aufgabe wird erfindungsgemäß dadurch gelöst, daß das Einstellelement mit einer Steuerung verbunden ist, die umschaltbar mindestens zwei Parametersätze zur Definition von Betriebsmodi vorgibt.

Eine zusätzliche Aufgabe der vorliegenden Erfindung besteht darin, eine Vorrichtung zur Durchführung einer Beatmung derart zu konstruieren, daß eine kompakte Steuereinheit bereitgestellt wird, die mit hoher Genauigkeit einen zu realisierenden Beatmungsparameter vorgibt.

Diese Aufgabe wird erfindungsgemäß dadurch gelöst, daß das Einstellelement als ein Proportionalventil ausgebildet ist, das elektrisch ansteuerbar und mit mindestens einem Piezo-Element zur Ausführung einer Stellbewegung versehen ist.

Es wird durch die Erfindung ein leistungsfähiges System bereitgestellt, daß unabhängig von einer externen Energieversorgung betriebsfähig ist. Die sensorische Überwachung der Patientenatmung vermeidet eine Schwächung von Restatmungsfunktionen des Patienten und durch die Umschaltbarkeit zwischen unterschiedlichen Parametersätzen ist ist möglich, kurzfristig und mit extrem geringer Fehlerwahrscheinlichkeit auch bei komplexen Anwendungssituationen eine optimale Beatmung durchzuführen.

Eine Versorgung der elektronischen Bauelemente dadurch unterstützt, daß zur Speisung elektrischer Funktionen ein Akumulator verwendet wird.

Eine schnelle Inbetriebnahme wird dadurch unterstützt, daß eine Umschaltbarkeit zwischen mindestens zwei Betriebsmodi vorgesehen ist.

Zur Abdeckung eines typischen Anwendungsbereiches wird vorgeschlagen, daß eine Umschaltbarkeit zwischen drei Betriebsmodi vorgesehen ist.

Eine typische Anwendung besteht darin, daß durch einen Parametersatz ein Betriebsmodus zur Versorgung eines Kleinkindes definiert wird.

Eine weitere Anwendung ist dadurch definiert, daß durch einen Parametersatz ein Betriebsmodus zur Versorgung eines Kindes definiert wird.

Ebenfalls besteht ein typischer Anwendungsbereich darin, daß durch einen Parametersatz ein Betriebsmodus zur Versorgung eines Erwachsenen definiert wird.

Zur Gewährleistung einer Einsatzfähigkeit im Außenbereich wird vorgeschlagen, daß ein Betriebstemperaturbereich im Intervall von minus 15 Grad Celsius bis plus 60 Grad Celsius bereitgestellt wird.

Eine typische meßtechnische Funktionserfassung wird dadurch ermöglicht, daß zur Überwachung der Atmungsfunktion des Patienten eine Druckmessung durchgeführt wird.

Eine andere Möglichkeit besteht darin, daß zur Überwachung der Atmungsfunktion des Patienten eine expiratorische Volumenmessung durchgeführt wird.

Ebenfalls ist daran gedacht, daß zur Überwachung der Atmungsfunktion des Patienten eine Kapnometrie durchgeführt wird.

Zur Vermeidung von Schädigungen des Atmungssystems des Patienten wird vorgeschlagen, daß eine Atemwegsdruckbegrenzung implementiert wird.

Zur Bereitstellung einer kompakten Ausführungsform, zur Verminderung des Montageaufwandes sowie zur Verringerung von Druckverlusten wird vorgeschlagen, daß Steuerventile zur Vorgabe der Beatmungsfunktionen innerhalb eines Halterungsblockes mit Strömungskanälen angeordnet sind.

Eine genaue Einstellmöglichkeit wird dadurch bereitgestellt, daß eine Umsetzung der Parametersätze in physikalische Ventileinstellungen mit Schrittmotoren durchgeführt wird.

Durch die Verwendung eines Proportionalventiles in Piezo-Technik als Einstellelement ist es möglich, bei kompakter Bauweise ein sehr genaues Stellelement bereitzustellen, das weitgehend hysteresefrei betrieben werden kann.

Zur Vervielfachung des verfügbaren Stellbereiches wird vorgeschlagen, daß das Piezo-Element aus einem Stapel von Piezo-Scheiben ausgebildet ist.

Eine typische Bauelementeanordnung besteht darin, daß die Piezo-Scheiben eine Materialkonsistenz derart aufweisen, daß bei Anlegen einer elektrischen Spannung ein Verkippen hervorrufbar ist.

Zur mechanischen Übersetzung der Stellbewegungen wird vorgeschlagen, daß das Piezo-Element mit einem Hebel gekoppelt ist.

Ein kompakter Aufbau wird dadurch unterstützt, daß das Einstellelement als ein Drehgelenk mit einer Drehachse im Flächenschwerpunkt ausgebildet ist.

Eine zuverlässige Konstruktion bei vergleichsweise geringem Bauelementepreis wird dadurch ermöglicht, daß die Piezo-Scheiben als Keramiklaminate ausgebildet sind.

Zur Vermeidung von Zugbelastungen des Stapels der Piezo-Scheiben wird vorgeschlagen, daß die gestapelten Piezo-Scheiben mit einer mechanischen Stapelvorspannung beaufschlagt sind.

In der Zeichnung sind Ausführungsbeispiele der Erfindung schematisch dargestellt. Es zeigen:
- Fig. 1:: eine Darstellung eines Beatmungsverlaufes bei assostierter Beatmung,
- Fig. 2:: ein Diagramm zur Veranschaulichung eines Druckverlaufes mit aufeinander folgenden Phasen von maschineller Ventilation und Spontanatmungsformen,
- Fig. 3:: eine Prinzipdarstellung eines Einstellelementes, das einen Stapel von Piezo-Elementen aufweist, die mit einem Hebel gekoppelt sind
**und**
- Fig. 4:: eine Prinzipdarstellung zur Veranschaulichung der Funktionsweise eines Stapels von scheibenförmigen Piezo-Elementen.

Die in den Zeichnungen dargestellten Beatmungsverläufe zeigen typische Effekte, die sich durch das nachfolgend beschriebene Verfahren und die zugehörige Vorrichtung hervorrufen lassen.

Es wird eine Betriebsfunktion bereitgestellt, mit der sowohl eine kontrollierte Beatmung als auch eine differenzierte Beatmung des Patienten mit assistierenden Beatmungsmodi möglich ist. Eine Vorwahl der gewünschten Atmungsparameter kann beispielsweise über Tastendruck erfolgen. Insbesondere ist daran gedacht, drei unterschiedliche Betriebsmodi vorzusehen, nämlich für die Beatmung von Kleinkindern, von Kindern und von Erwachsenen.

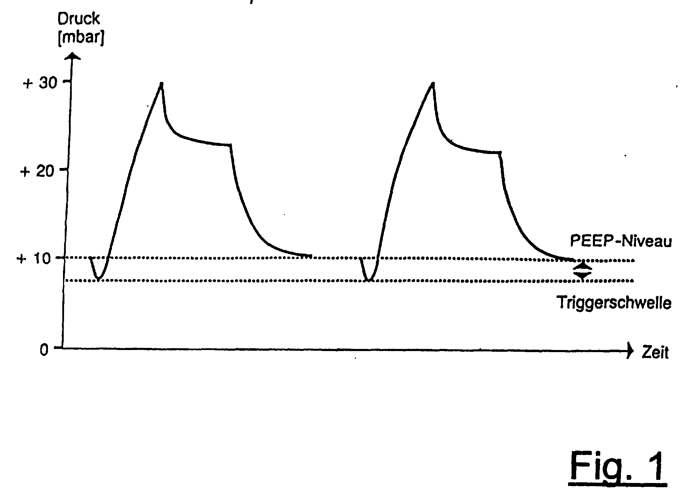

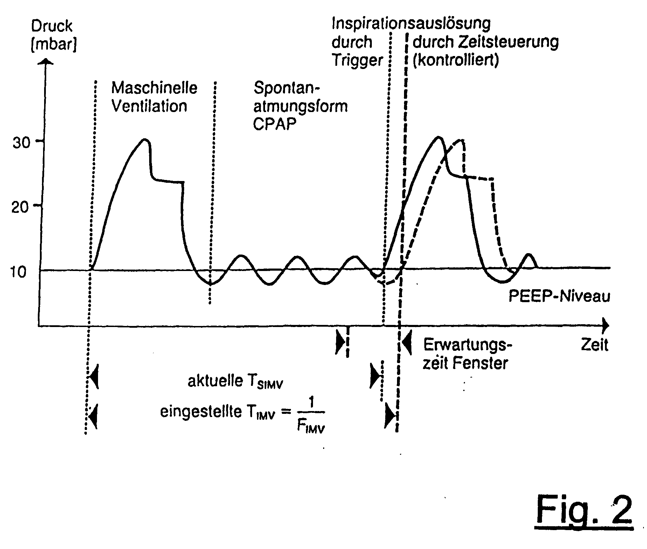

Insbesondere ist auch daran gedacht, durch eine Parametervorgabe eine Beeinflussung der in Figur 1 und Figur 2 dargestellten Beatmungsdruckkurven durchzuführen. Insbesondere ist dabei auch daran gedacht, unterschiedliche Druckbegrenzungen vorzusehen, um Spitzendruckverläufe im Bereich der Atemwege des Patienten zu reduzieren. Eine weitere Maßnahme besteht darin, den jeweiligen Volumenstrom der Luftförderung an den jeweils vorliegenden Grad der Lungenfüllung des Patienten anzupassen. Hierbei kann insbesondere berücksichtigt werden, daß zu einem Beginn der Lungenfüllung mit geringem Druck ein relativ großer Volumenstrom zweckmäßig ist und daß mit steigendem Füllgrad der Lunge durch eine Verminderung des Volumenstromes ein ungünstiger Druckanstieg vermieden werden kann. Dieser ungünstige Effekt kann durch den beschriebenen deszellerierenden Druckverlauf vermieden werden.

Zur Anpassung der Beatmungsfunktionen des Beatmungsgerätes an die jeweils vorliegende Atmungsfähigkeit des Patienten ist es möglich, die erforderliche sensorische Erfassung beispielsweise durch Druckmessung, expiratorische Volumenmessung und Kapnometrie durchzuführen.

Gemäß einer vereinfachten Version wird lediglich ein Druck über ein Manometer angezeigt, insbesondere ist jedoch daran gedacht, ein Display vorzusehen, daß beispielsweise die Druckkurve, den Atemwegsspitzendruck, das expiratorische Volumen, die Kapnografenfunktion, den Beatmungsmodus und ggf. vorliegende Alarme anzeigt. Grundsätzlich ist es auch denkbar, zusätzliche Funktionen oder lediglich eine Teilmenge der vorgenannten Funktionen anzuzeigen bzw. darzustellen.

Gemäß einer weiteren Ausführungsvariante ist daran gedacht, über eine Datenschnittstelle eine Auswertung von Gerätedaten zu unterstützen. Darüber hinaus ist es möglich, über eine oder mehrerer Datenschnittstellen externe Meßwerte, beispielsweise eine EKG-Kurve, auf dem Gerätedisplay darzustellen.

Eine Beeinflussung der Atmungsverläufe des Patienten ist in unterschiedlichen Verläufen möglich. Beispielsweise zeigt Figur 1 einen Atmungsverlauf, bei dem die Atmung nicht gegen den normalen Umgebungsdruck, sondern gegen einen voreingestellten Druckwert erfolgt. Im dargestellten Beispiel sind dies 10 mbar. Eine Triggerschwelle des Gerätes ist beim dargestellten Verlauf auf 2,5 mbar eingestellt.

Ein weiterer Atmungsverlauf ist in Figur 2 abgebildet. Es liegt eine Mischform zwischen einer Spontanatmung und einer maschinellen Beatmung vor. Es besteht die Möglichkeit, den Atemhub der Beatmungseinrichtung mit dem Atemzug des Patienten zu synchronisieren. Der maschinelle Atemhub wird ausgelöst, wenn der Patient nach dem Ende einer Spontanatmungsphase eine neuerliche Einatemanstrengung unternimmt und damit einen Trägerimpuls auslöst. Die Auslösung des maschinellen Atemhubes kann ggf. einige Sekunden früher erfolgen, als es dem Ablauf der betreffenden Zeit entspricht. Hieraus kann eine Frequenzerhöhung folgen.

Der dargestellte intervallartige Beatmungsverlauf kann beispielsweise verwendet werden, um Patienten, die über einen langen Zeitraum beatmet wurden, wieder an eine vollständige Eigenatmung heranzuführen. Ebenfalls ist aber möglich, einen derartigen Verlauf bei der Durchführung von Langzeitbeatmungen zu verwenden, da die Kreislaufbelastung des Patienten hierdurch reduziert werden kann. Darüber hinaus bleibt der Spontanatemantrieb des Patienten weitgehend erhalten. Durch die maschinelle Beatmung wird somit im wesentlichen eine Mindestventilation gewährleistet.

Durch die Voreinstellung der Betriebsmodi können unterschiedliche Parameter beeinflußt werden. Beispielsweise ist es möglich, daß Atmungsvolumen, die Atmungsfrequenz, den maximalen Druck sowie das Verhältnis I:E zu beeinflussen. Die Einstellung kann beispielsweise durch Ansteuerung von Schrittmotoren erfolgen, die durch elektronische Regeleinheiten einzeln angesprochen werden können. Eine Auslösung der jeweiligen Betriebsmodi kann durch kleine Taster, beispielsweise im Bereich einer Tastaturfolie, erfolgen, bei der jeweils zugehörige Piktogramme für den jeweils vorgesehenen Benutzerkreis angeordnet sind.

Die durch diese Wahl des Betriebsmodus vor- eingestellten Werte können im Bereich der visuellen Anzeige dargestellt werden. Zusätzlich ist es möglich, diese vordefinierten Parameter durch Verwendung von manuellen Reglern zu verändern. Ein Benutzer wird somit nicht gezwungen, die voreingestellten Werte zu verwenden, sondern die Voreinstellung dient lediglich dazu, ohne Zeitverzögerung eine betriebsfähige Grundfunktion zu gewährleisten. Nach Bereitstellung dieser Grundfunktion kann eine Optimierung erfolgen.

Konstruktiv ist insbesondere daran gedacht, die Pneumatischen Bauelemente getrennt von den elektrischen Bauelementen anzuordnen. Hierdurch wird eine Erhöhung der Betriebssicherheit hervorgerufen, da die elektronischen Komponenten außerhalb einer Sauerstoffatmosphäre angeordnet sind.

Eine weitere konstruktive Verbesserung kann durch die Realisierung der Verrichtung in Blocktechnik erfolgen. Es wird hierdurch eine schlauchlose Verbindung der Einzelventile bereitgestellt und eine geringe Leckrate erreicht. Innerhalb des Pneumatikblockes vorgesehene Entlüftungskanäle können gebündelt in einen Hauptentlüftungskanal eingeleitet werden, der aus dem Gehäuse herausführt. Durch die vorgeschlagene Entlüftung wird somit unter Berücksichtigung der Entlüftungsvorgänge der Pneumatikventile vermieden, daß die Sauerstoffkonzentration innerhalb des Gerätes erhöht wird.

Ein typischer Temparaturbereich, in dem eine Funktionsfähigkeit gegeben ist, liegt zwischen minus 15 Grad Celsius und plus 60 Grad Celsius.

Die Ventilsteuerungen können mit Hilfe von Proportionalventilen mit Piezotechnik realisiert werden. Der verwendete Pneumatikblock wird vorzugsweise aus einem leichten Werkstoff, beispielsweise Aluminium oder Kunststoff, gefertigt. Die Verbindung der einzelnen Ventile untereinander erfolgt durch Bohrungskanäle.

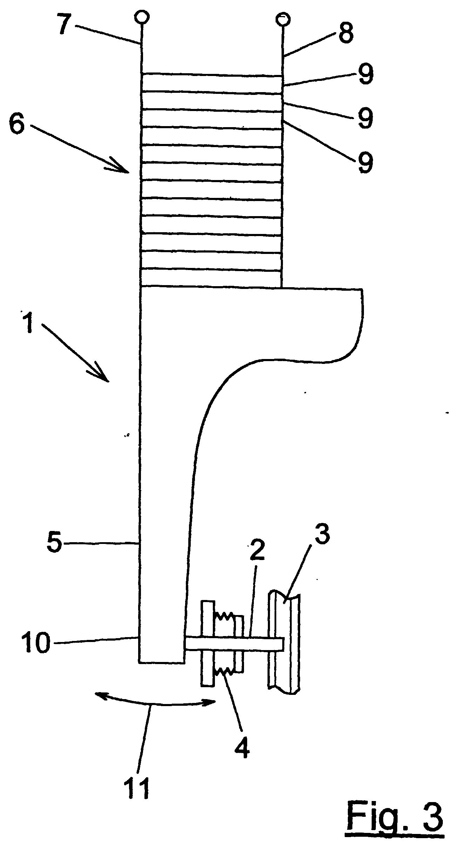

Fig. 3 zeigt den prinzipiellen Aufbau eines Einstellelementes (1), das ein Ventilelement (2) positioniert, das im Bereich eines Strömungskanales (3) angeordnet ist und diesen vorgebbar versperrt oder ganz beziehungsweise teilweise freigibt. Das Ventilelement (2) kann beispielsweise derart realisiert sein, daß von einem Einstellelement (4) eine Grundposition vorgegeben wird. Entsprechend den jeweiligen Anwendungsanforderungen kann die Grundposition beispielsweise einer vollständig geöffneten oder eine vollständig geschlossenen Ventilposition entsprechen. Das Einstellelement (4) kann als elektrisches Bauelement, beispielsweise unter Einbeziehung einer Wegmessung, ausgebildet sein. Grundsätzlich ist auch eine mechanische Realisierung, beispielsweise als Feder, möglich.

Das Ventilelement (2) ist mit einem Hebel (5) gekoppelt, der von einem Piezo-Element (6) positionierbar ist. Über Anschlüsse (7,8) ist das Piezo-Element (6) elektrisch steuerbar. Insbesondere ist daran gedacht, das Piezo-Element (6) aus einer Vielzahl von Piezo-Scheiben (9) auszubilden. Entsprechend der Anzahl der Piezoscheiben (9) wird die bei Einwirkung der elektrischen Energie verursachte Verformung der Piezo-Scheiben (9) vervielfacht.

Die Piezo-Scheiben (9) können beispielsweise als Keramiklaminate ausgebildet sein. Bei Anlegen einer elektrischen Spannung führen die Piezo-Scheiben (9) eine Kippbewegung durch, wobei der Bewegungsweg durch den Hebel (5) vergrößert auf das Ventilelement (2) übertragen wird. Ein dem Piezo-Element (6) abgewandtes Hebelende (10) kann entsprechend der jeweils angelegten elektrischen Steuerspannung innerhalb eines Schwenkbereiches (11) positioniert werden. Ein typischer Stellweg beträgt 6 mm. Bei der Auslegung des Hebels ist zu berücksichtigen, daß der Vergrößerung des Stellweges eine entsprechende Verkleinerung der Stellkraft gegenübersteht.

Piezo-Scheiben (9) sind vorzugsweise druckbelastbar ausgebildet. Größere Zugbeanspruchungen können hingegen zu Materialbeanspruchungen führen. Es kann deshalb vorgesehen werden, eine mechanische Vorspannung der Piezo-Scheiben (9) zu realisieren, so daß vorzugsweise im gesamten Arbeitsbereich eine resultierende Druckbeanspruchung gegeben ist. Eine typische Ausführungsform wird derart gewählt, daß von der Kombination aus den Piezo-Scheiben (9) und dem Hebel (5) eine Schwenkbewegung um einen Flächenschwerpunkt herum durchgeführt wird.

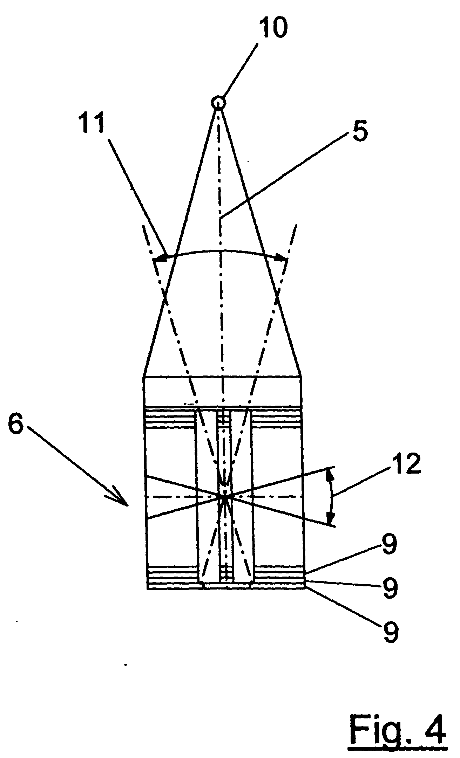

Fig. 4 veranschaulicht zusätzlich die Wirkungsweise einer Mehrzahl von gestapelten Piezo-Scheiben (9). Es ist insbesondere noch einmal verdeutlicht, wie eine Winkelverkippung der Piezo-Scheiben (9) in einem Bewegungsbereich (12) in eine Schwenkbewegung des schematisiert dargestellten Hebels (5) innerhalb des Schwenkbereiches (11) transformiert wird.

Die Einstellgenauigkeit des Ventilelementes (2) kann durch einen Betrieb des Einstellelementes (1) innerhalb eines geschlossenen Regelkreises weiter verbessert wer den. Als Meßparameter kann beispielsweise über eine kontinuierliche Messung die sich ergebende Strömungsgeschwindigkeit beziehungsweise die Durchflußmenge des Beatmungsgases, oder ein Beatmungsdruck am Ausgang des Beatmungsgerätes erfaßt werden. Das von dem oder den Sensoren bereitgestellte elektrische Meßsignal wird der Regelungselektronik zur Verfügung gestellt. Eine Verwendung derartiger Einstellelemente (1) in Piezo-Technik ist sowohl für mobile als auch für stationäre Beatmungsgeräte möglich.

Physikalisch bedingt treten bei Piezo-Elementen (6) Steuerabweichungen durch Nichtlinearitäten, Hystereseeffekte sowie Drifterscheinungen auf. Sämtliche Effekte lassen sich jedoch durch geschlossene Regelkreise vollständig kompensieren, so daß eine extrem hohe Stellgenauigkeit erreichbar ist. Beispielsweise ist es möglich, direkt in den Stapel der Piezo-Scheiben (9) ein Wegmeßsystem zu integrieren, das die konkret vorliegende Dehnung erfaßt und über eine zugeordnete Regelung gegebenenfalls eine Korrektur des Stellsignals verursacht. Dies hat eine entsprechende Anpassung der Betriebsspannung des Piezo-Elementes (6) zur Folge.

## Patentansprüche

1. Verfahren zum Betrieb eines Beatmungsgerätes, das bei einer mobilen Versorgung eines Patienten angewendet wird, dadurch gekennzeichnet, daß sensorisch mindestens ein Atmungsparameter des Patienten erfaßt und zur Gerätesteuerung derart ausgewertet wird, daß im wesentlichen eine detektierte Atmungsfehlfunktion substituiert und eine eigene Atmungsaktivität des Patienten berücksichtigt wird und daß eine Betriebsenergie mindestens teilweise von einer Druckgasspeisung bereitgestellt wird.

2. Verfahren nach Anruch 1, dadurch gekennzeichnet, daß zur Speisung elektrischer Funktionen ein Akumulator verwendet wird.

3. Verfahren nach Anspruch 1 oder 2, dadurch gekennzeichnet, daß eine Umschaltbarkeit zwischen mindestens zwei Betriebsmodi vorgesehen ist.

4. Verfahren nach Anspruch 3, dadurch gekennzeichnet, daß eine Umschaltbarkeit zwischen drei Betriebsmodi vorgesehen ist.

5. Verfahren nach Anspruch 3 oder 4, dadurch gekennzeichnet, daß durch einen Parametersatz ein Betriebsmodus zur Versorgung eines Kleinkindes definiert wird.

6. Verfahren nach Anspruch 5, dadurch gekennzeichnet, daß durch einen Parametersatz ein Betriebsmodus zur Versorgung eines Kindes definiert wird.

7. Verfahren nach Anspruch 5 oder 6, dadurch gekennzeichnet, daß durch einen Parametersatz ein Betriebsmodus zur Versorgung eines Erwachsenen definiert wird.

8. Verfahren nach einem der Ansprüche 1 bis 7 dadurch gekennzeichnet, daß ein Betriebstemperaturbereich im Intervall von minus 15 Grad Celsius bis plus 60 Grad Celsius bereitgestellt wird.

9. Verfahren nach einem der Ansprüche 1 bis 8, dadurch gekennzeichnet, daß zur Überwachung der Atmungsfunktion des Patienten eine Druckmessung durchgeführt wird.

10. Verfahren nach einem der Ansprüche 1 bis 9, dadurch gekennzeichnet, daß zur Überwachung der Atmungsfunktion des Patienten eine expiratorische Volumenmessung durchgeführt wird.

11. Verfahren nach einem der Ansprüche 1 bis 10, dadurch gekennzeichnet, daß zur Überwachung der Atmungsfunktion des Patienten eine Kapnometrie durchgeführt wird.

12. Verfahren nach einem der Ansprüche 1 bis 11, dadurch gekennzeichnet, daß eine Atemwegsdruckbegrenzung implementiert wird.

13. Vorrichtung zur Durchführung einer Beatmung eines Patienten, die als eine mobile Einrichtung ausgebildet ist und die mindestens ein Einstellelement zur Vorgabe mindestens eines Betriebsparameters aufweist, dadurch gekennzeichnet, daß das Einstellelement mit einer Steuerung verbunden ist, die umschaltbar mindestens zwei Parametersätze zur Definition von Betriebsmodi vorgibt.

14. Vorrichtung nach Anspruch 13, dadurch gekennzeichnet, daß Einstellungen für drei Betriebsmodi vorgesehen sind.

15. Vorrichtung nach Anspruch 13 oder 14, dadurch gekennzeichnet, daß ein Parametersatz zur Steuerung der Beatmungsfunktionen für ein Kleinkind vorgesehen ist.

16. Vorrichtung nach einem der Ansprüche 13 bis 15, dadurch gekennzeichnet, daß ein Parametersatz zur Steuerung der Beatmungsfunktionen für ein Kind vorgesehen ist.

17. Vorrichtung nach einem der Ansprüche 1 bis 16, dadurch gekennzeichnet, daß der Parametersatz als Einzelparameter mindestens Voreinstellwerte für MV, f, Pmax und I:E enthält.

18. Vorrichtung nach einem der Ansprüche 13 bis 17, dadurch gekennzeichnet, daß ein Parametersatz zur Steuerung der Beatmungsfunktionen für einen Erwachsenen vorgesehen ist.

19. Vorrichtung nach einem der Ansprüche 13 bis 18, dadurch gekennzeichnet, daß Steuerventile zur Vorgabe der Beatmungsfunktionen innerhalb eines Halterungsblockes mit Strömungskanälen angeordnet sind.

20. Vorrichtung nach einem der Ansprüche 13 bis 19, dadurch gekennzeichnet, daß für eine Umsetzung der Parametersätze in physikalische Ventileinstellungen Schrittmotoren vorgesehen sind.

21. Verfahren zum Betrieb eines Beatmungsgerätes, das bei einer mobilen Versorgung eines Patienten angewendet wird, dadurch gekennzeichnet, daß ein Volumenstrom des Atemgases und ein Beatmungsdruck derart aufeinander abgestimmt werden, daß bei zunehmender Lungenfüllung des Patienten eine Reduzierung des Volumenstromes zur Druckbegrenzung durchgeführt wird.

22. Vorrichtung zur Durchführung einer Beatmung eines Patienten, die eine Atemgasversorgung, ein Kopplungselement zum Anschluß an den Patienten sowie mindestens ein Einstellelement zur Beeinflussung mindestens eines Beatmungsparameters aufweist, dadurch gekennzeichnet, daß das Einstellelement (1) als ein Proportionalventil ausgebildet ist, das elektrisch ansteuerbar und mit mindestens einem Piezo-Element (6) zur Ausführung einer Stellbewegung versehen ist.

23. Vorrichtung nach Anspruch 22, dadurch gekennzeichnet, daß das Piezo-Element (6) aus einem Stapel von Piezo-Scheiben (9) ausgebildet ist.

24. Vorrichtung nach Anspruch 22 oder 23, dadurch gekennzeichnet, daß die Piezo-Scheiben (9) eine Materialkonsistenz derart aufweisen, daß bei Anlegen einer elektrischen Spannung ein Verkippen hervorrufbar ist.

25. Vorrichtung nach einem der Ansprüche 22 bis 24, dadurch gekennzeichnet, daß das Piezo-Element (6) mit einen Hebel (5) gekoppelt ist.

26. Vorrichtung nach einem der Ansprüche 22 bis 25, dadurch gekennzeichnet, daß das Einstellelement (1) als ein Drehgelenk mit einer Drehachse im Flächenschwerpunkt ausgebildet ist.

27. Vorrichtung nach einem der Ansprüche 22 bis 26, dadurch gekennzeichnet, daß die Piezo-Scheiben (9) als Keramiklaminate ausgebildet sind.

28. Vorrichtung nach einem der Ansprüche 22 bis 27, dadurch gekennzeichnet, daß die gestapelten Piezo-Scheiben (9) mit einer mechanischen Stapelvorspannung beaufschlagt sind.
